(19) Europäisches Patentamt
European Patent Office
Office européen des brevets

(11) EP 0 777 286 B1

(12) **EUROPÄISCHE PATENTSCHRIFT**

(45) Veröffentlichungstag und Bekanntmachung des Hinweises auf die Patenterteilung:
**04.03.1998 Patentblatt 1998/10**

(51) Int Cl.6: **H01M 6/50**, H01M 10/40, A61N 1/378

(21) Anmeldenummer: **95250273.0**

(22) Anmeldetag: **08.11.1995**

(54) **Batteriebetriebenes implantierbares Gerät**

Implantable battery powered apparatus

Appareil implantable alimenté par une batterie

(84) Benannte Vertragsstaaten:
**DE FR GB IT SE**

(43) Veröffentlichungstag der Anmeldung:
**04.06.1997 Patentblatt 1997/23**

(73) Patentinhaber: **LITRONIK Batterietechnologie GmbH & Co.**
**01796 Pirna (DE)**

(72) Erfinder:
• **Staub, Roland**
**D-01796 Pirna (DE)**
• **Fehrmann, Gerd**
**D-01796 Pirna (DE)**

• **Wolf, Rüdiger, Dr.**
**D-01734 Rabenau (DE)**
• **Fischer, Thomas, Dr.**
**D-01796 Pirna (DE)**
• **Heimer, Hartmut**
**D-01796 Pirna (DE)**

(74) Vertreter: **Christiansen, Henning, Dipl.-Ing.**
**Patentanwalt**
**Pacelliallee 43/45**
**14195 Berlin (DE)**

(56) Entgegenhaltungen:
**US-A- 3 757 793**     **US-A- 3 757 795**
**US-A- 4 543 304**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft ein batteriebetriebenes implantierbares medizinisches Gerät mit einer Batterie, einer Meßvorrichtung zur Bestimmung des Ladezustands der Batterie durch Messung der Leerlaufspannung und einem elektrischen Verbraucher gemäß dem Oberbegriff des Anspruchs 1.

Es sind batteriebetriebene implantierbare medizinische Geräte bekannt, die eine Meßvorrichtung zur Bestimmung des Ladezustands der Batterie aufweisen.

In der US-PS 3,757,793 ist ein durch eine Batterie betriebener Herzschrittmacher beschrieben, bei dem eine Bestimmung des Ladezustands der Batterie durch Messung der Leerlaufspannung vorgesehen ist. Die Leerlaufspannung nimmt dabei während der Entladung stufenweise bis auf Null ab.

Zu Beginn der Entladung weist die vorbekannte Batterie einen Bereich mit einer erhöhten Anfangsspannung auf. Nach einer Entladung der Batterie in Höhe von ca. 10% der Gesamtenergie sinkt die Leerlaufspannung nahezu sprunghaft auf die Betriebsspannung ab. Die Leerlaufspannung bleibt auf diesem Niveau annähernd konstant, bis die Batterie ca. 90% ihrer Gesamtenergie durch Entladung abgegeben hat. Anschließend sinkt die Leerlaufspannung nahezu sprunghaft auf eine Endspannung ab, die deutlich unterhalb der Betriebsspannung liegt, aber zum Betrieb des Geräts ausreicht. Diese Endspannung ist annähernd konstant, bis die Batterie ihre gesamte nutzbare Restenergie verloren hat. Dann sinkt die Leerlaufspannung ebenfalls nahezu sprunghaft auf Null ab.

Die vorbekannte Batterie ermöglicht es auf einfache Weise, den Ladezustand der Batterie zu ermitteln. Hierzu wird die Leerlaufspannung gemessen und mit den Werten der Anfangsspannung, der Betriebsspannung und der Endspannung verglichen.

Es kann auf diese Weise sichergestellt werden, daß nur vollständig geladene Batterien implantiert werden.

Darüberhinaus ist es möglich, das bevorstehende Ende der Lebensdauer der Batterie zu einem Zeitpunkt zu erkennen, in dem die verbleibende Restenergie zur Aufrechterhaltung des Betriebs für einen zur Durchführung eines Therapievorgangs notwendigen Mindestzeitraum noch ausreicht. Während dieses Zeitraums kann z.B. die Batterie ausgetauscht werden. Es ist deshalb möglich, die Energie der Batterie nahezu vollständig auszunutzen, da das Ende der Lebensdauer zuverlässig vorhersehbar ist und somit diesbezüglich keine Sicherheitsreserven notwendig sind.

Bei der vorbekannten Anordnung tritt jedoch ein Problem auf.

Zur Messung der Leerlaufspannung und zur Bestimmung des Ladezustands aus der gemessenen Spannung ist zumindest im implantierten Zustand des Geräts eine elektronische Schaltung erforderlich. Diese Schaltung benötigt jedoch in der Regel eine symmetrische Spannungsversorgung. Es ist also eine Spannungsversorgung notwendig, die ein Bezugspotential, sowie zwei - bezogen auf das Bezugspotential - unterschiedliche Polaritäten liefert. Dies wird von der bekannten Anordnung nicht geleistet.

Der Erfindung liegt deshalb insbesondere die Aufgabe zugrunde, ein batteriebetriebenes implantierbares medizinisches Gerät zu schaffen, bei dem die Batterie sowohl die Spannungsversorgung eines elektrischen Verbrauchers als auch die symmetrische Spannungsversorgung einer zur Bestimmung des Ladezustands der Batterie verwendeten Meßvorrichtung ermöglicht. Darüberhinaus soll der hierzu notwendige zusätzliche apparative Aufwand im Hinblick auf die Implantation des Geräts in den menschlichen Körper hinsichtlich des zusätzlichen Gewichts und des zusätzlichen Bauvolumens möglichst gering sein.

Die Aufgabe wird mit den im kennzeichnenden Teil des Anspruchs 1 angegebenen Maßnahmen gelöst.

Die Erfindung schließt die technische Lehre ein, daß eine Batterie eine Reihenschaltung mehrerer Zellen mit drei ein symmetrisches Spannungssystem bildenden Spannungsabgriffen aufweist und eine von dem symmetrischen Spannungssystem versorgte Meßvorrichtung die maximal zwischen den drei Spannungsabgriffen auftretende Spannung auswertet.

Die erfindungsgemäße Anordnung besteht im wesentlichen aus einer Batterie, einer Meßvorrichtung zur Bestimmung des Ladezustands der Batterie und einem elektrischen Verbraucher, beispielsweise dem Impulsgeber eines Herzschrittmachers.

Die in der erfindungsgemäßen Anordnung enthaltene Batterie gibt - wie die in der US-PS 3,757,793 vorbeschriebene Batterie - in Abhängigkeit von der verbleibenden Restenergie abgestufte diskrete Spannungswerte ab.

In der Batterie ist eine Reihenschaltung mehrerer Zellen vorgesehen, wodurch sich die Spannungen der einzelnen Zellen addieren. Der Abstand zwischen den diskreten Spannungswerten der Batterie nimmt dadurch ebenfalls zu, wodurch die Unterscheidung der diskreten Spannungswerte und damit die Bestimmung des Ladezustands erleichtert wird.

Die Reihenschaltung weist jeweils eine frei Anode und eine freie Katode auf. An der freien Anode ist ein erster Spannungsabgriff und an der freien Katode ein zweiter Spannungsabgriff vorgesehen, so daß ein erstes und ein zweites Potential und damit die gesamte über der Batterie abfallende Spannung abgreifbar ist.

Der elektrische Verbraucher ist zur Spannungsversorgung mit dem ersten und dem zweiten Spannungsabgriff verbunden und verfügt dadurch über die gesamte über der Batterie abfallende Spannung als Versorgungsspannung.

An einer anderen, in der Reihenschaltung zwischen den äußeren Elektroden liegenden Elektrode ist ein dritter Spannungsabgriff vorgesehen. Das Potential dieses dritten Spannungsabgriffs liegt zwischen dem Potential des ersten und des zweiten Spannungsabgriffs und dient als Bezugspotential. Hierdurch entsteht ein symmetrisches Spannungsystem zur Spannungsversorgung der Meßvorrichtung.

Die Meßvorrichtung wertet die Batteriespannung aus und erzeugt entsprechend dem Ladezustand ein Batteriezustandssignal. Hierzu mißt die Meßvorrichtung den gesamten über der Batterie abfallenden Spannungshub und vergleicht den gemessenen Wert mit den diskreten Spannungswerten, die den Ladezustand der Batterie wiederspiegeln.

Die erfindungsgemäße Anordnung erzielt also drei Hauptwirkungen.

Erstens steht für den elektrischen Verbraucher die gesamte an der Batterie abfallende Spannung zur Verfügung.

Zweitens wird ein symmetrisches Spannungssystem zur Versorgung der Meßvorrichtung bereitgestellt.

Drittens wertet die Meßvorrichtung die gesamte über der Batterie abfallenden Spannung aus und erreicht dadurch eine größtmögliche Genauigkeit.

Mit zunehmender Genauigkeit der Meßvorrichtung sinken die Anforderungen an den Abstand der diskreten Spannungswerte. Bei einer großen Genauigkeit der Meßvorrichtung können so die diskreten Spannungswerte dichter zusammenliegen.

Hierdurch ist es vorteilhaft möglich, die Endspannung nahe an die Betriebsspannung heranzulegen und dadurch die mit der verringerten Endspannung verbundenen negativen Folgen zu minimieren.

Zum Erreichen des beschriebenen stufenförmigen Spannungsverlaufs mit mehreren diskreten Spannungswerten ist ein relativ hoher konstruktiver Aufwand hinsichtlich der Zusammensetzung der Elektroden und des Elektrolyten notwendig, wobei die Erreichung des stufenförmigen Spannungsverlaufs und die Kapazitätsmaximierung teilweise nicht vereinbar sind. Durch die Erhöhung der Genauigkeit der Meßvorrichtung und die daraus resultierenden geringeren Anforderungen an den Abstand der diskreten Spannungswerte der Batterie wird so vorteilhaft der konstruktive Spielraum erhöht.

In einer bevorzugten Ausführungsform der Erfindung besteht die Meßschaltung aus einem Addierer, der die Spannung zwischen dem ersten Potential und dem Bezugspotential sowie zwischen dem Bezugspotential und dem zweiten Potential addiert.

Auf diese Weise wird vorteilhaft die gesamte über der Batterie abfallende Spannung ausgewertet.

Aus dem Ausgangssignal der Meßschaltung erzeugt ein Schwellwertglied das Batteriezustandssignal. Hierzu weist das Schwellwertglied einen oder mehrere Schwellwerte auf. In Abhängigkeit von der Lage des Ausgangssignals der Meßschaltung relativ zu dem oder den Schwellwerten wird das Batteriezustandssignal erzeugt.

In einer bevorzugten Ausführungsform der Erfindung weist das Schwellwertglied drei Schwellwerte auf.

Der erste Schwellwert liegt zwischen der Anfangsspannung und der Betriebsspannung der Batterie. Ein Überschreiten des ersten Schwellwertes kennzeichnet eine nahezu vollständig geladene Batterie.

Der zweite Schwellwert liegt zwischen der Betriebsspannung und der Endspannung der Batterie. Liegt das Ausgangssignal der Meßschaltung zwischen dem ersten und dem zweiten Schwellwert, so befindet sich die Batterie in einem normalen Entladungsbereich, d.h. die Batterie ist zwar nicht mehr vollständig geladen, aber ausreichend für einen sicheren Betrieb.

Der dritte Schwellwert liegt zwischen der Endspannung und Null. Liegt das Ausgangssignal der Meßschaltung zwischen dem zweiten und dem dritten Schwellwert, so befindet sich die Batterie in einer Endphase, in der die Batterie kurz vor ihrer völligen Erschöpfung steht.

Ein Unterschreiten des dritten Schwellwertes kennzeichnet eine vollständig entladene Batterie.

Das von dem Schwellwertglied erzeugte Batteriezustandssignal weist also vier mögliche Zustände auf, die den Entladezustand der Batterie kennzeichnen: voll, fast voll, fast leer und leer.

Eine Variante der Erfindung sieht eine Telemetrieübertragung des Ausgangssignals der Meßschaltung oder des Batteriezustandssignals an eine außerhalb des Körpers befindliche Empfangseinheit vor. Hierzu weist die Meßvorrichtung eine Sende-/Empfangseinheit auf. Die Übertragung kann beispielsweise drahtlos durch elektromagnetische Wellen erfolgen. Der Empfang erfolgt vorzugsweise durch Auflegen eines Antennenelements auf die Körperoberfläche in der Nähe der Sendeeinheit. Hierdurch kann die erforderliche Sendeleistung und damit der Energieverbrauch des Senders niedrig gehalten werden.

In einer Variante der Erfindung weist die Sende-/Empfangseinheit zwei Modi auf. Im normalen Betrieb arbeitet die Sende-/Empfangseinheit als Empfänger. Wird von einer außerhalb des Körpers befindlichen Sendeeinheit ein Aktivierungssignal gesendet, so schaltet die Sende-/Empfangseinheit in den Sendemodus um und überträgt das Batteriezustandssignal oder das Ausgangssignal der Meßschaltung an eine außerhalb des Körpers befindliche Empfangseinheit. Anschließend wird wieder in den Empfangsmodus zurückgeschaltet. Auf diese Weise wird vorteilhaft der Energieverbrauch minimiert, da der während des Sendebetriebs relativ hohe Energieverbrauch nur dann anfällt, wenn dies tatsächlich erforderlich ist.

Durch die Telemetrieübertragung kann der Batteriezustand einer in einem implantierten Gerät befindlichen Batterie in vorteilhafter Weise nicht-invasiv überprüft werden.

In einer anderen Variante der Erfindung weist der elektrische Verbraucher eine Steuereinheit auf, die das Batteriezustandssignal auswertet und in Abhängigkeit von dem Ladezustand der Batterie den Stromverbrauch steuert.

Dies kann beispielsweise dadurch geschehen, daß ein Herzschrittmacher die Impulsrate und/oder die Impulsamplitude senkt, wenn das Batteriezustandssignal eine fast leere Batterie signalisiert. Hierdurch wird die Lebensdauer der Anordnung verlängert.

In einer Variante der Erfindung ist das Gehäuse der Batterie elektrisch leitend mit dem dritten Spannungsabgriff verbunden, der somit auf Massepotential liegt. Die bei einem einfachen Isolationsfehler maximal auftretende Fehlerspannung wird dadurch - bei einem mittig zwischen dem ersten und dem zweiten Spannungsabgriff angeordneten dritten Spannungsabgriff - auf die Hälfte der Gesamtspannung der Batterie begrenzt.

Andere vorteilhafte Weiterbildungen der Erfindung sind in den Unteransprüchen gekennzeichnet bzw. werden nachstehend zusammen mit der Beschreibung der bevorzugten Ausführungen der Erfindung anhand der Figuren näher dargestellt. Es zeigen:

Figur 1     als Ausführungsbeispiel der Erfindung eine Anordnung mit einer Batterie, einer Meßschaltung, einem Schwellwertglied sowie einem elektrischen Verbraucher als Blockschaltbild,

Figur 2     eine bevorzugte Ausführungsform der in Figur 1 dargestellten Meßschaltung als Schaltbild,

Figur 3     eine weitere Ausführungsform der in Figur 1 dargestellten Meßschaltung als Schaltbild,

Figur 4     eine bevorzugte Ausführungsform der in Figur 1 dargestellten Batterie detailliert in perspektivischer Darstellung sowie

Figur 5     eine weitere Ausführungsform der in Figur 1 dargestellten Batterie detailliert in Querschnittsdarstellung.

Die Batterie weist zwei in Reihe geschaltete Zellen 1, 2 auf, wobei die Katode der ersten Zelle 1 und die Anode der zweiten Zelle 2 durch ein Verbindungsstück elektrisch leitend miteinander verbunden sind. An der Anode der ersten Zelle 1 ist ein erster Spannungsabgriff vorgesehen. An der Katode der zweiten Zelle 2 ist ein zweiter Spannungsabgriff vorgesehen. Auf diese Weise ist die gesamte über der Batterie abfallende Spannung abgreifbar.

Der Verbraucher 6 ist mit dem ersten und dem zweiten Spannungsabgriff elektrisch leitend verbunden und verfügt somit über die gesamte an der Batterie abfallende Spannung.

An dem Verbindungsstück zwischen den Zellen 1, 2 ist ein dritter Spannungsabgriff 3 vorgesehen, an dem ein Bezugspotential abgreifbar ist. Zusammen mit den an dem ersten und dem zweiten Spannungsabgriff abgreifbaren Potentialen steht auf diese Weise eine symmetrische Spannungsversorgung für die Meßvorrichtung 7 zur Verfügung.

Der dritte Spannungsabgriff 3 ist mit dem Gehäuse der Batterie verbunden. Dadurch ist die bei einem einfachen Isolationsfehler auftretende Kurzschlußspannung auf die Spannung der schadhaften Zelle 1, 2 begrenzt, die ungefähr halb so groß ist wie die gesamte Batteriespannung.

Die Meßvorrichtung 7 besteht im wesentlichen aus einer Meßschaltung 4 und einem Schwellwertglied 5.

Die Meßschaltung 4 überlagert die auf das Bezugspotential bezogene Änderung des ersten und des zweiten Potentials additiv und erzeugt daraus ein Ausgangssignal, das dem über der gesamten Batterie abfallenden Spannungshub entspricht. Bei einer Vergrößerung des gesamten Spannungshubs wird auch das Ausgangssignal größer; bei einer Verringerung des gesamten Spannungshubs wird auch das Ausgangssignal kleiner.

Das Schwellwertglied 5 weist drei Schwellwerte auf und erzeugt ein Batteriezustandssignal, das von der Lage des Ausgangssignals der Meßschaltung relativ zu den Schwellwerten abhängt. Der erste Schwellwerte liegt zwischen der Anfangsspannung und der Betriebsspannung der Batterie; der zweite Schwellwert liegt zwischen der Betriebsspannung und der Endspannung und der dritte Schwellwert liegt zwischen der Endspannung und Null. Liegt die Batteriespannung oberhalb des ersten Schwellwertes, so signalisiert das Batteriezustandssignal eine vollständig gefüllte Batterie; sinkt die Batteriespannung unter den ersten Schwellwert, so signalisiert das Batteriezustandssignal eine volle Batterie; sinkt die Batteriespannung weiter unter den zweiten Schwellwert, so signalisiert das Batteriezustandssignal eine fast leere Batterie; bei einem Absinken der Batteriespannung unter den dritten Schwellwert signalisiert das Batteriezustandssignal schließlich eine vollständig entleerte Batterie.

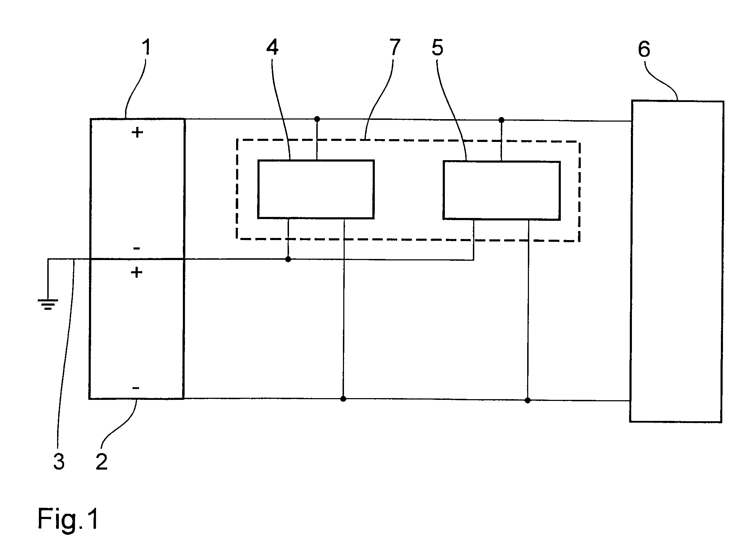

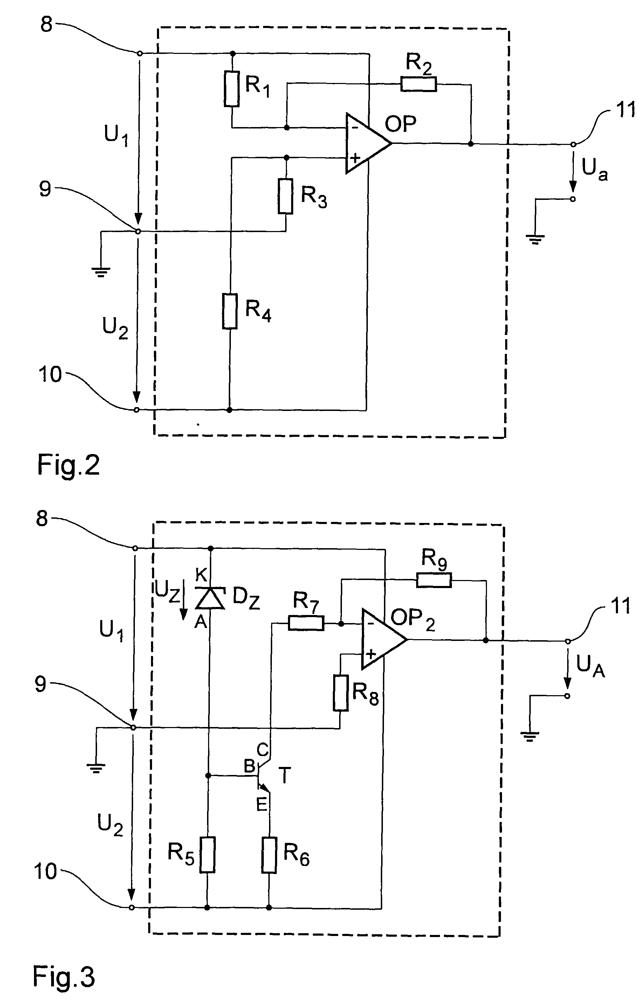

Figur 2 zeigt ein Ausführungsbeispiel der in Figur 1 dargestellten Meßschaltung 4 detailliert als Schaltbild.

Die Meßschaltung 4 besteht im wesentlichen aus einem Operationsverstärker OP und vier Widerständen $R_1$, $R_2$, $R_3$ und $R_4$.

Der Operationsverstärker arbeitet in der dargestellten Anordnung als Subtrahierer, der die Spannung $U_1$ von der Spannung $(-U_2)$ subtrahiert, also hier als Addierer betrieben wird.

Die Spannung $U_1$ ist die Spannung zwischen dem ersten Spannungsabgriff 8 und dem dritten Spannungsabgriff 9, während die Spannung $U_2$ die Spannung zwischem dem dritten Spannungsabgriff 9 und dem zweiten Spannungsabgriff 10 ist. Der Addierer bildet also die Summe der über den beiden Zellen abfallenden Spannungen und erzeugt ein der gesamten Batteriespannung proportionales Ausgangssignal $U_A$.

Für die Ausgangsspannung $U_A$ des Addierers gilt:

$$U_A = k \cdot (-U_2 - U_1) = -k \cdot (U_1 + U_2) \text{ mit } k = \frac{R_2}{R_1}$$

4

Zur ordnungsgemäßen Funktion des Addierers müssen die Widerstände $R_1$, $R_2$, $R_3$ und $R_4$ die Koeffizientenbedingung erfüllen:

$$\frac{R_2}{R_1} = \frac{R_3}{R_4}$$

Durch eine Variation des Verstärkungsfaktors k läßt sich der Pegel des Ausgangssignals $U_A$ der Meßschaltung 4 an den Eingang des Schwellwertgliedes 5 anpassen.

Figur 3 zeigt ein weiteres Ausführungsbeispiel der in Figur 1 dargestellten Meßschaltung 4 detailliert als Schaltbild.

Die Schaltung überlagert - wie die in Figur 2 dargestellte Schaltung - die auf das Bezugspotential bezogene Änderung des ersten und des zweiten Potentials.

Am Eingang der Meßschaltung ist ein Pegelwandler vorgesehen, bestehend aus der Zenerdiode $D_Z$ und dem Widerstand $R_5$. Der Pegelwandler bildet die Summe der Spannungen $U_1$ und $U_2$ und verringert diese um die konstante Spannung $U_Z$ der Zenerdiode $D_Z$. An dem Widerstand $R_5$ fällt deshalb die Spannung $U = U_1 + U_2 - U_Z$ ab. Es ist also der gesamte Spannungshub der Batterie an dem Widerstand $R_5$ abgreifbar.

Dieser Spannungshub wird über den Transistor T dem Operationsverstärker OP2 zugeführt.

Der Operationsverstärker OP2 verstärkt den Spannungshub und gibt an dem Ausgang 11 ein Ausgangssignal $U_A$ ab, das annähernd proportional dem gesamten Spannungshub ist.

Durch eine Variation des Verhältnisses der Widerstände $R_9$ und $R_7$ läßt sich der Verstärkungsfaktor des Operationsverstärkers OP2 einstellen und damit der Pegel der Ausgangsspannung UA an den Eingang des Schwellwertgliedes 5 anpassen. Durch eine Vergrößerung des Quotienten aus $R_9$ und $R_7$ läßt sich die Ausgangsspannung $U_A$ anheben und durch eine Veringerung des Quotienten entsprechend absenken.

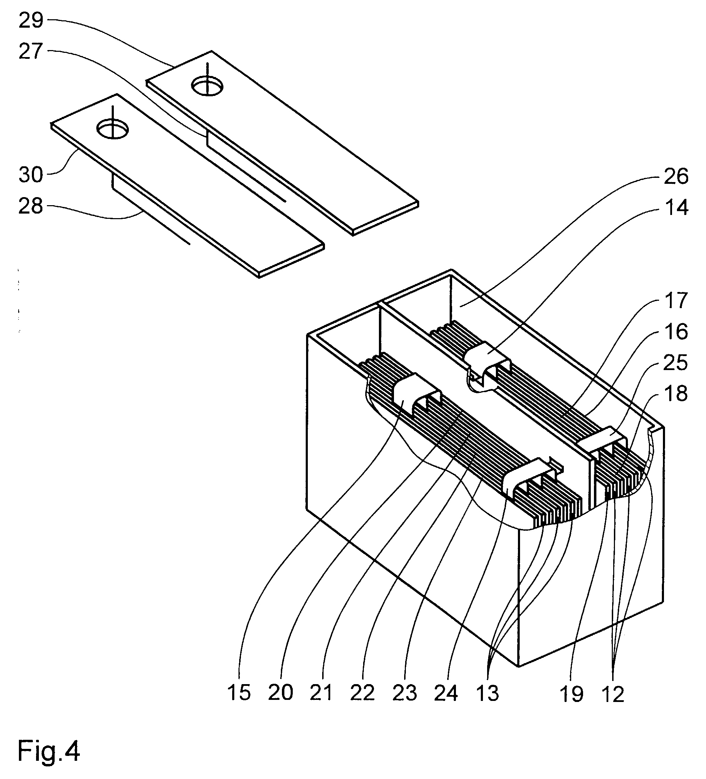

Figur 4 zeigt ein Ausführungsbeispiel der in Figur 1 dargestellten Batterie mit zwei Zellen und entsprechend zwei Elektrodenpaketen, die in jeweils einem Fach eines Gehäuses 15 untergebracht sind.

Jedes der beiden Elektrodenpakete weist als Elektroden drei Katodenplatten 12 bzw. 13 und vier Anodenplatten 16, 17, 18, 19 bzw. 20, 21, 22, 23 gleicher Größe auf, die in der Weise parallel angeordnet sind, daß jede Katodeplatte 12, 13 von jeweils zwei Anodenplatten 16, 17, 18, 19, 20, 21, 22, 23 benachbart ist. Ebenso denkbar sind Elektrodenpakete mit je 4 Katoden und 5 Anoden usw..

Die Katodenplatten 12, 13 und die Anodenplatten 16, 17, 18, 19, 20, 21, 22, 23 haben eine rechteckige Form mit einer über die Tiefe im wesentlichen einheitlichen Breite. Hierdurch ist es vorteilhaft möglich, die Elektrodenpakete vorzumontieren und im montierten Zustand in die Fächer in dem Batteriegehäuse 26 zu schieben.

Die Katodenplatten 12, 13 enthalten eine Mischung aus $CrO_x$ und $MnO_2$ mit $PbCrO_4$, $PbMo_4$ oder PbO oder eine Mischung von $MnO_2$ mit PbO, Acetylenruß, Graphit und Teflondispersion oder Komponenten hiervon.

Die Anodenplatten 16, 17, 18, 19, 20, 21, 22, 23 bestehen vorzugsweise aus metallischem Lithium.

Die zu einem Elektrodenpaket gehörigen Anodenplatten 16, 17, 18, 19, bzw. 20, 21, 22, 23 sind jeweils durch einen Anodenverbinder 24 bzw. 25 elektrisch leitend miteinander verbunden, während die zu einem Elektrodenpaket gehörigen Katodenplatten 12 bzw. 13 jeweils durch einen Katodenverbinder 14 bzw. 15 elektrisch leitend miteinander verbunden sind.

Die Kapazität einer Zelle ist dabei durch die Größe der Oberfläche der Elektrodenplatten 12, 13, 16, 17, 18, 19, 20, 21, 22, 23 und die Zahl der parallelgeschalteten Platten beeinflußt.

Das Batteriegehäuse 26 besteht im wesentlichen aus einem wannenförmigen Unterteil, einer Trennwand, die zwei Fächer in dem Gehäuse abteilt und zwei Deckeln 29, 30, die die beiden Fächer abschließen.

Beide Elektrodenpakete sind von einer Tasche aus nicht leitfähigem Plastmaterial umhüllt und dadurch gegen das Gehäuse 15 und die Trennwand elektrisch isoliert.

Das Gehäuse, die Deckel 29, 30 und die Trennwand bestehen aus einem elektrisch leitfähigen Material und sind elektrisch leitend miteinander verbunden. Die Trennwand bildet eine Verbindung der beiden Zellen, indem der Katodenverbinder 14 des ersten Elektrodenpakets und der Anodenverbinder 24 des zweiten Elektrodenpakets mittels einer Punktschweißung mit der Trennwand verbunden sind. Da die Trennwand elektrisch leitend mit dem Gehäuse 15 verbunden ist, liegt der Spannungsabgriff auf Massepotential. Hierdurch wird die maximal gegen Masse auftretende Spannung der Batterie auf die Hälfte des gesamten Spannungsabfalls begrenzt.

Der Anodenverbinder 25 des ersten Elektrodenpakets und der Katodenverbinder 15 des zweiten Elektrodenpakets sind elektrisch gegeneinander und das Gehäuse 26 isoliert und werden als Pin durch jeweils eine Glasdurchführung in den Deckeln 29, 30 herausgeführt.

Die Batterie ist als ganzes hermetisch dicht.

Das Gehäuse 26 ist in beiden Fächern mit einem Elektrolyten gefüllt, wobei der Füllstand so bemessen ist, daß die Elektrodenplatten 12, 13, 16, 17, 18, 19, 20, 21, 22, 23 ganz abgedeckt sind. Als Elektrolyt wird vorzugsweise eine Mischung aus Propylencarbonat (PC), Ethylencarbonat (EC), Dimethoxyethan (DME) und $LiClO_4$ verwendet. Jedoch kann die Batterie auch mit anderen Elektrolyten (z.B. Methylformiat, Tetrahydrofuran) und anderen Leitsalzen (z.B. $LiAsF_6$, LiPF6) gefüllt werden.

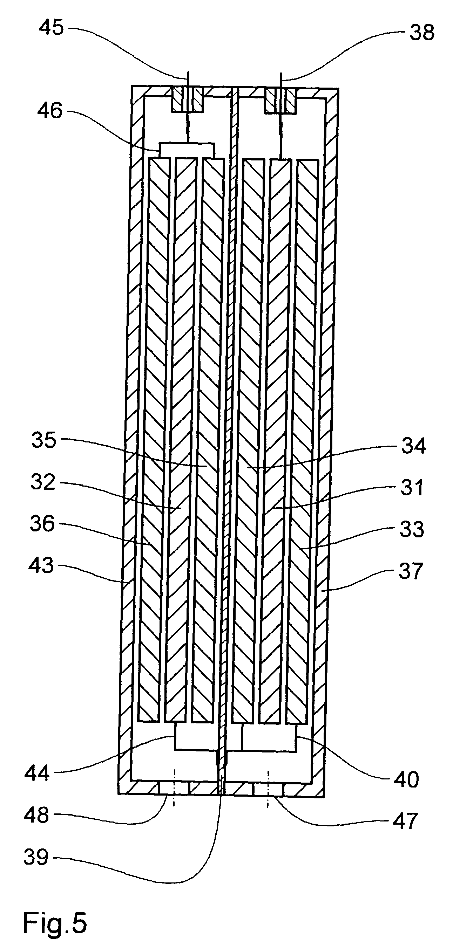

Figur 5 zeigt eine Batterie mit zwei Batteriezellen und entsprechend zwei Elektrodenpaketen, die jeweils in einem Fach eines Gehäuses untergebracht sind.

Jedes Elektrodenpaket weist als Elektroden jeweils eine Katodenplatte 31 bzw. 32 und zwei Anodenplatten 33, 34 bzw. 35, 36 gleicher Größe auf, wobei die Katodenplatten 31 bzw. 32 jeweils mittig zwischen den Anodenplatten 33, 34 bzw. 35, 36 und parallel zu diesen angeordnet sind.

Der Abstand zwischen den Elektrodenplatten 31, 32, 33, 34, 35, 36 liegt in der gleichen Größenordnung wie die Dicke der Elektrodenplatten 31, 32, 33, 34, 35, 36, wodurch zum einen ein gutes Funktionieren der bei der Auf- und Entladung ablaufenden elektrochemischen Prozesse und zum anderen eine optimale Ausnutzung des zur Verfügung stehenden Raums gewährleistet wird.

Das hier beschriebene Ausführungsbeispiel unterscheidet sich von dem in Figur 4 dargestellten Ausführungsbeispiel durch die Art der Verkapselung und damit zusammenhängend die Art der Montage der Batterie.

Das Gehäuse besteht im wesentlichen aus zwei Halbschalen 37 und 43 und einer Trennwand 39.

Sowohl die Halbschalen 37, 43 als auch die Trennwand 39 bestehen aus einem elektrisch leitfähigen Material und sind elektrisch leitend miteinander verbunden. Die Trennwand 39 bildet eine elektrische Verbindung der beiden Zellen, indem der Anodenverbinder 40 des zweiten Elektrodenpakets und ein an der Katodenplatte 32 des ersten Elektrodenpakets angebrachtes Kontaktstück 44 mittels einer Punktschweißung elektrisch leitend mit der Trennwand 39 verbunden sind, wodurch eine Reihenschaltung der beiden Batteriezellen mit einem Spannungsmittenabgriff entsteht.

Durch die Ausbildung des Gehäuses in Form von zwei Halbschalen 37, 43 ist es vorteilhaft möglich, zunächst die Elektrodenpakete vorzumontieren und auf der Trennwand 39 zu befestigen und anschließend nur noch die Halbschalen 37, 43 an den Umfangsrändern der Trennwand 39 auf diese aufzulegen und abzudichten. Die Durchführung der Schweißarbeiten zur Kontaktierung von Trennwand 39 und Anodenverbinder 40 bzw. Katodenplatte 32 ist deshalb produktionstechnisch einfacher.

Ein an der Katodenplatte 31 des zweiten Elektrodenpakets angebrachter Kontakt und der Anodenverbinder 46 des ersten Elektrodenpakets sind elektrisch isoliert und über eine Glasdurchführung 38 bzw. 45 herausgeführt.

In jeder Halbschale 37, 43 befindet sich darüberhinaus jeweils eine verschließbare Öffnung 47, 48 zum Einfüllen des Elektrolyten.

Der Elektrolyt besteht vorzugsweise aus einer Mischung aus Propylencarbonat (PC), Ethylencarbonat (EC), Dimetoxyethan (DME) und $LiClO_4$, aber es sind auch andere bekannte Kombinationen denkbar.

Die Erfindung beschränkt sich in ihrer Ausführung nicht auf die vorstehend angegebenen bevorzugten Ausführungsbeispiele. Vielmehr ist eine Anzahl von Varianten denkbar, welche von der. dargestellten Lösung auch bei grundsätzlich anders gearteten Ausführungen Gebrauch macht.

**Patentansprüche**

1. Batteriebetriebenes implantierbares medizinisches Gerät, mit einer Batterie, einer Meßvorrichtung (7) zur Bestimmung des Ladezustands der Batterie durch Messung der Leerlaufspannung und einem elektrischen Verbraucher (6),
   wobei die Leerlaufspannung der Batterie bereits in einem Bereich einen erhöhten Abfall aufweist, in dem die verbleibende Restenergie zur Aufrechterhaltung des Betriebs für einen zur Durchführung eines Therapievorgangs notwendigen Mindestzeitraum noch ausreicht,
   **dadurch gekennzeichnet,**

   daß die Batterie eine Reihenschaltung mehrerer Zellen (1, 2) aufweist,

   daß zum Abgriff eines ersten Potentials an der freien Anode der Batterie ein erster Spannungsabgriff (8, 27, 45) und zum Abgriff eines zweiten Potentials an der freien Katode (10) der Batterie ein zweiter Spannungsabgriff (10, 28, 38) vorgesehen ist,

   daß der elektrische Verbraucher (6) zur Versorgung mit der gesamten über der Batterie abfallenden Spannung jeweils mit dem ersten und dem zweiten Spannungsabgriff elektrisch leitend verbunden ist,

   daß zum Abgriff eines Bezugspotentials und zur Versorgung der Meßvorrichtung (7) mit einer symmetrischen Spannungsversorgung ein dritter Spannungsabgriff (3, 9) an einer in der Reihenschaltung zwischen den äußeren Elektroden liegenden Elektrode vorgesehen ist,

   daß zur Messung der gesamten Batteriespannung eine an ihren Meßeingängen mit dem ersten und dem zweiten Spannungsabgriff elektrisch leitend verbundene Meßschaltung (4) vorgesehen ist, die einen Pegelwandler und/oder einen Addierer aufweist,

   daß die Meßvorrichtung (7) zur Erzeugung eines Batteriezustandssignals ein an dem Ausgang der Meßschaltung (4) angeschlossenes Schwellwertglied (5) mit zumindest einem Schwellwert aufweist,

   daß die Meßschaltung (4) und das Schwellwertglied (5) jeweils einen Eingang für einen Bezugspunkt aufweisen, der mit dem dritten Spannungsabgriff (3, 9) elektrisch leitend verbunden ist,

daß die Meßschaltung (4) und das Schwellwertglied (5) zur Versorgung mit einer zu dem dritten Spannungsabgriff (3) symmetrischen Spannung jeweils mit dem ersten und dem zweiten Spannungsabgriff elektrisch leitend verbunden sind.

2. Batteriebetriebenes Gerät nach Anspruch 1, **dadurch gekennzeichnet,**

daß die Meßschaltung (4) zur schaltungstechnischen Synthese eines Addierers vier Widerstände ($R_1$, $R_2$, $R_3$, $R_4$) sowie einen Operationsverstärker (OP) mit einem P-Eingang (+), einem N-Eingang (-) einem Ausgang (11) und zwei Spannungsversorgungseingängen aufweist,

daß der erste Widerstand ($R_1$) den N-Eingang (-) des Operationsverstärker (OP) mit dem ersten Spannungsabgriff (8) verbindet,

daß der zweite Widerstand ($R_2$) zur Erzeugung einer Rückkopplung den N-Eingang (-) des Operationsverstärkers (OP) mit dessen Ausgang (11) verbindet,

daß der dritte Widerstand ($R_3$) den P-Eingang (+) des Operationsverstärkers (OP) mit dem dritten Spannungsabgriff (9) verbindet,

daß der vierte Widerstand ($R_4$) den P-Eingang (+) des Operationsverstärkers (OP) mit dem zweiten Spannungsabgriff (10) verbindet,

daß die Spannungsversorgungseingänge des Operationsverstärkers mit dem ersten (8) und dem zweiten (10) Spannungsabgriff verbunden sind,

daß zur Erfüllung der Koeffizientenbedingung das Verhältnis der Widerstandswerte des zweiten ($R_2$) und des ersten ($R_1$) Widerstands sowie des dritten ($R_3$) und des vierten ($R_4$) Widerstands im wesentlichen gleich ist.

3. Batteriebetriebenes Gerät nach Anspruch 1, **dadurch gekennzeichnet,**

daß die Meßschaltung (4) zur Anpassung ihres Ausgangssignals an das Schwellwertglied (5) einen Verstärker und zum Abgriff des gesamten über der Batterie abfallenden Spannungshubs sowie zur Anpassung an den Verstärker einen Pegelwandler aufweist,

daß der Pegelwandler im wesentlichen aus einer Zener-Diode ($D_Z$) und einem ersten Widerstand ($R_3$) besteht, wobei die Katode (K) der Zener-Diode ($D_Z$) mit dem ersten Spannungsabgriff (8) verbunden ist und der erste Widerstand ($R_5$) die Anode (A) der Zener-Diode ($D_Z$) mit dem zweiten Spannungsabgriff (10) verbindet,

daß zum Abgriff des gesamten an der Batterie abfallenden Spannungshubs und zur Anpassung an den Eingang eines Verstärkers ein in Emitterschaltung betriebener Transistor (T) vorgesehen ist, wobei die Basis (B) mit der Anode (A) der Zener-Diode ($D_Z$) und der Emitter (E) durch einen zweiten Widerstand ($R_6$) mit dem zweiten Spannungsabgriff (10) verbunden ist,

daß der Verstärker im wesentlichen aus einem einem dritten ($R_7$), vierten ($R_8$) und fünften ($R_9$) Widerstand sowie einem Operationsverstärker (OP2) mit einem N-Eingang (-), einem P-Eingang (+), einem Ausgang sowie zwei Spannungsversorgungseingängen besteht,

daß der N-Eingang (-) des Operationsverstärkers (OP2) durch den dritten Widerstand ($R_7$) mit dem Kollektor (C) des Transistors (T) verbunden ist,

daß der P-Eingang (+) des Operationsverstärkers (OP2) durch den vierten Widerstand ($R_8$) mit dem dritten Spannungsabgriff (9) verbunden ist,

daß zur Erreichung einer Rückkopplung der Ausgang des Operationsverstärkers (OP2) durch den fünften Widerstand ($R_9$) mit dem N-Eingang (-) verbunden ist,

daß die Spannungsversorgungseingänge des Operationsverstärkers (OP2) mit dem ersten (8) und dem zweiten (10) Spannungsabgriff verbunden sind.

4. atteriebetriebenes Gerät nach einem der vorhergehenden Ansprüche, **dadurch gekennzeichnet,** daß ein Gehäuse (26) vorgesehen ist, das elektrisch leitend mit dem dritten Spannungsabgriff (9) verbunden ist.

5. Batteriebetriebenes Gerät nach einem der vorhergehenden Ansprüche, **dadurch gekennzeichnet,** daß die Meßvorrichtung (7) zur leitungslosen oder leitungsgebundenen Übertragung des Batteriezustandssignals an eine außerhalb des Geräts befindliche Empfangseinheit und/oder zum Empfang eines Aktivierungssignals von einer außerhalb des Geräts befindlichen Sendeeinheit eine Sende-/Empfangseinheit aufweist.

6. Batteriebetriebenes Gerät nach einem der vorhergehenden Ansprüche, **dadurch gekennzeichnet,** daß der elektrische Verbraucher (6) zu einer von dem Ladezustand der Batterie abhängigen Beeinflussung seines Stromverbrauchs eine Steuereinheit aufweist, an deren Eingang das Batteriezustandssignal anliegt.

7. Batteriebetriebenes Gerät nach einem der vorhergehenden Ansprüche, **dadurch gekennzeichnet,**

   daß die Batterie ein im montierten Zustand hermetisch dichtes Gehäuse (26) mit zwei durch eine Trennwand (39) elektrisch zu einer Reihenschaltung verbundenen, aber räumlich getrennten Zellen aufweist,

   daß jede Zelle ein Elektrodenpaket enthält, das einander abwechselnd benachbart angeordnete, ebene oder gekrümmte Anodenplatten (16, 17, 18, 19, 20, 21, 22, 23, 33, 34, 35, 36) und Katodenplatten (12, 13, 31, 32) aufweist,

   daß jeweils in beiden Zellen die Katodenplatten (12, 13, 31, 32) miteinander durch einen Katodenverbinder (14, 15) und die Anodenplatten (16, 17, 18, 19, 20, 21, 22, 23, 33, 34, 35, 36) miteinander durch einen Anodenverbinder (24, 25, 40, 46) elektrisch leitend verbunden sind,

   daß der Anodenverbinder (25, 40) der ersten Zelle mit dem ersten Spannungsabgriff (27) und der Katodenverbinder (15) der zweiten Zelle mit dem zweiten Spannungsabgriff (28) elektrisch leitend verbunden ist,

   daß der Anodenverbinder (24) der zweiten Zelle und der Katodenverbinder (14) der ersten Zelle elektrisch leitend mit der Trennwand (39), dem Gehäuse (26) und dem dritten Spannungsabgriff (9) verbunden sind.

8. Batteriebetriebenes Gerät nach einem der vorhergehenden Ansprüche, **dadurch gekennzeichnet,** daß die Elektrodenpakete zur Isolierung gegen das Gehäuse (26) und die Trennwand (39) in einer aus Kunststoff bestehenden Tasche stecken.

9. Batteriebetriebenes Gerät nach einem der vorhergehenden Ansprüche, **dadurch gekennzeichnet,** daß das Gehäuse zwei Halbschalen (37, 43) aufweist.

10. Batteriebetriebenes Gerät nach einem der vorhergehenden Ansprüche, **dadurch gekennzeichnet,** daß die Katodenplatten (12, 13, 31, 32) eine Mischung aus $CrO_x$ mit x zwischen 2,5 und 2,7 oder $CrO_x$ und $MnO_2$ mit $PbCrO_4$, $PbMoO_4$ oder PbO oder eine Mischung von $MnO_2$ mit PbO enthalten.

11. Batteriebetriebenes Gerät nach einem der vorhergehenden Ansprüche, **dadurch gekennzeichnet,** daß die Katodenplatten (12, 13, 31, 32) an der Oberfläche eine Beschichtung aufweisen, die ein Bindemittel und einen Leitzusatz enthält.

12. Batteriebetriebenes Gerät nach einem der vorhergehenden Ansprüche, **dadurch gekennzeichnet,** daß die Katodenplatten (12, 13, 31, 32) eine Porosität von 30 bis 50 Prozent aufweisen.

13. Batteriebetriebenes Gerät nach einem der vorhergehenden Ansprüche, **dadurch gekennzeichnet,** daß die Anodenplatten (16, 17, 18, 19, 20, 21, 22, 23, 33, 34, 35, 36) im wesentlichen aus Lithium bestehen.

14. Batteriebetriebenes Gerät nach einem der vorhergehenden Ansprüche, **dadurch gekennzeichnet,** daß die Batterie einen Elektrolyten enthält, der aus 20 bis 60 Prozent Ethylencarbonat (EC) und 5 bis 20 Prozent Propylencarbonat (PC= und 30 bis 70 Prozent Dimethoxyethan (DME) besteht.

15. Batteriebetriebenes Gerät nach einem der vorhergehenden Ansprüche, **dadurch gekennzeichnet,** daß der Elektrolyt $LiClO_4$ in einer Konzentration von 1,0 bis 1,5 $^{Mol}/_l$ enthält.

16. Batteriebetriebenes Gerät nach einem der vorhergehenden Ansprüche, **dadurch gekennzeichnet,** daß das Gehäuse (26) im wesentlichen aus CrNi-Stahl, Molybdän, Titan oder Nickel besteht.

**Claims**

1. A battery-operated implantable medical device, comprising a battery, a measuring device (7) to determine the battery's state of charge by measuring the open circuit voltage and an electrical consumer (6), wherein the battery's open circuit voltage already shows an increased drop in a region in which the remaining energy is still sufficient to maintain operation for a minimum period of time necessary for carrying out a therapeutic process, characterised in that

   the battery comprises a plurality of cells (1, 2) connected in series,

   that in order to tap a first potential at the battery's free anode, a first voltage tap (8, 27, 45) is provided, and that in order to tap a second potential at the battery's free cathode (10), a second voltage tap (10, 28, 38) is provided,

that the electrical consumer (6) is connected in an electrically-conductive manner to the first and second voltage taps in order to be supplied by the total voltage drop across the battery,

that a third voltage tap (3, 9), located at an electrode in the series circuit between the outer electrodes, is provided to tap a reference voltage level and to supply the measuring device (7) with a symmetrical voltage supply,

that a measurement circuit (4) is provided, connected in an electrically-conductive manner at its measuring inputs to the first and second voltage taps to determine the total battery voltage, which has a level converter and/or an adder,

that the measuring device (7) has a threshold value element (5) for generating a battery status signal, which has at least one threshold value, and is connected at the output of the measurement circuit (4),

that the measurement circuit (4) and the threshold value element (5) each have an input for a reference point which is connected in an electrically-conductive manner to the third voltage tap (3, 9), and

that the measurement circuit (4) and the threshold value element (5) are each connected in an electrically-conductive manner to the first and second voltage taps in order to be supplied by a voltage symmetrical to the third voltage tap.

2. A battery-operated device according to claim 1, characterised in that the measurement circuit (4) comprises four resistances ($R_1$, $R_2$, $R_3$, $R_4$) and an operational amplifier (OP) with a P input (+), an N input (-), an output (11) and two voltage supply inputs in order to synthesise an adder,

that the first resistance ($R_1$) connects the N input (-) of the operational amplifier (OP) with the first voltage tap (8),

that the second resistance ($R_2$) connects the N input (-) and the output (11) of the operational amplifier (OP) in order to create a feedback loop,

that the third resistance ($R_3$) connects the P input (+) of the operational amplifier (OP) with the third voltage tap (9),

that the fourth resistance ($R_4$) connects the P input (+) of the operational amplifier (OP) with the second voltage tap (10),

that the voltage supply inputs of the operational amplifier are connected with the first (8) and second (10) voltage taps,

and that in order to fulfil the coefficient conditions, the relationship between the resistance values of the second ($R_2$) and the first ($R_1$) resistance, and between the third ($R_3$) and the fourth ($R_4$) resistance is substantially the same.

3. A battery-operated device according to claim 1, characterised in that the measurement circuit (4) comprises an amplifier to match its output signal to the threshold value element (5), and has a level converter to allow it to tap the whole voltage range dropped across the battery, and also to interface with the amplifier,

that the level converter substantially consists of a Zener diode ($D_Z$) and a first resistance ($R_3$), where the cathode (K) of the Zener diode ($D_Z$) is connected to the first voltage tap (8) and the first resistance ($R_5$) connects the anode (A) of the Zener diode ($D_Z$) with the second voltage tap (10),

that a transistor (T) is provided, operating in emitter connection, for tapping the entire voltage range dropped at the battery and for interfacing with the input of an amplifier, where the base (B) is connected to the anode (A) of the Zener diode ($D_Z$) and the emitter (E), is connected to the second voltage tap (10) via a second resistance ($R_6$),

that the amplifier substantially consists of a third ($R_7$), fourth ($R_8$) and fifth ($R_9$) resistance, and an operational amplifier (OP2) with an N input (-), a P input (+) an output and two voltage supply inputs,

that the N input (-) of the operational amplifier (OP2) is connected to the collector (C) of the transistor (T) via the third resistance ($R_7$),

that the P input (+) of the operational amplifier (OP2) is connected to the third voltage tap (9) via the fourth resistance ($R_8$),

that the output of the operational amplifier (OP2) is connected to the N input (-) via the fifth resistance ($R_9$) to create a feedback loop,

and that the voltage supply inputs of the operational amplifier (OP2) are connected to the first (8) and second (10) voltage taps.

4. A battery-operated device according to one of the preceding claims,
characterised in that a casing (26) is provided which is connected in an electrically-conductive manner to the third voltage tap (9).

5. A battery-operated device according to one of the preceding claims,
characterised in that the measuring device (7) comprises a transmitter/receiver unit for cable-free or cable-transmitted conveyance of the battery status signal to a receiver unit located outside the device and/or for receipt of an activation signal from a transmitter unit located outside the device.

6. A battery-operated device according to one of the preceding claims,
characterised in that the electrical consumer (6) comprises a control unit for influencing its current consumption, dependent on the state of charge of the battery, and that the battery status signal is present at the input of this control unit.

7. A battery-operated device according to one of the preceding claims,
characterised in that the battery has a casing (26), which is hermetically-sealed in its assembled state, with two cells which are electrically connected in series by a partition (39) but are spatially separated,

that each cell contains an electrode package which has alternating flat or rounded anode plates (16, 17, 18, 19, 20, 21, 22, 23, 33, 34, 35, 36) and cathode plates (12, 13, 31, 32) arranged next to each other,

that in each cell the cathode plates (12, 13, 31, 32) are connected in an electrically-conductive manner by a cathode connector (14, 15) and the anode plates (16, 17, 18, 19, 20, 21, 22, 23, 33, 34, 35, 36) are connected in an electrically-conductive manner by an anode connector (24, 25, 40, 46),

that the anode connector (25, 40) of the first cell is connected in an electrically-conductive manner to the first voltage tap (27) and the cathode connector (15) of the second cell is connected in an electrically-conductive manner to the second voltage tap (28),

and that the anode connector (24) of the second cell and the cathode connector (14) of the first cell are connected to the partition (39), the casing (26) and the third voltage tap (9) in an electrically-conductive manner.

8. A battery-operated device according to one of the preceding claims,
characterised in that the electrode packages are received in a pouch of synthetic material to insulate them from the casing (26) and the partition (39).

9. A battery-operated device according to one of the preceding claims,
characterised in that the casing comprises two half-shells (37, 43).

10. A battery-operated device according to one of the preceding claims,
characterised in that the cathode plates (12, 13, 31, 32) contain a mixture of $CrO_x$, where x is between 2.5 and 2.7, or $CrO_x$ and $MnO_2$ with $PbCrO_4$, $PbMoO_4$ or PbO, or a mixture of $MnO_2$ with PbO.

11. A battery-operated device according to one of the preceding claims,
characterised in that the cathode plates (12, 13, 31, 32) have a coating on their surface which contains a binding agent and a conductive supplement.

12. A battery-operated device according to one of the preceding claims,
characterised in that the cathode plates (12, 13, 31, 32) have a porosity of between 30 and 50 per cent.

13. A battery-operated device according to one of the preceding claims,
characterised in that the anode plates (16, 17, 18, 19, 20, 21, 22, 23, 33, 34, 35, 36) substantially consist of lithium.

14. A battery-operated device according to one of the preceding claims,
characterised in that the battery contains an electrolyte which consists of between 20 and 60 per cent ethylene carbonate (EC) and between 5 and 20 per cent propylene carbonate (PC) and 30 to 70 per cent dimethoxyethane (DME).

15. A battery-operated device according to one of the preceding claims,
characterised in that the electrolyte contains $LiClO_4$ at a concentration of between 1.0 and 1.5 $^{Mol}/_l$.

16. A battery-operated device according to one of the preceding claims,
characterised in that the casing (26) substantially consists of CrNi steel, molybdenum, titanium or nickel.

## Revendications

1. Appareil médical implantable, alimenté par batterie, comportant une batterie, un dispositif de mesure (7) pour déterminer l'état de charge de la batterie par mesure de la tension à vide, et un récepteur électrique (6),

dans le cas duquel la tension à vide de la batterie présente déjà dans une zone une décroissance accrue où l'énergie résiduelle qui subsiste suffit encore pour maintenir le fonctionnement pendant un temps minimal nécessaire pour l'exécution d'un processus thérapeutique,

caractérisé par le fait

que la batterie présente un circuit de série de plusieurs cellules (1, 2),

que, pour saisir un premier potentiel à l'anode libre de la batterie est prévue une première prise de tension (8, 27, 45) et, pour saisir un second potentiel à la cathode libre (10) de la batterie, est prévue une seconde prise de tension (10, 28, 38),

que, pour son alimentation en la totalité de la tension fournie par la batterie, le récepteur électrique (6) est relié, avec conduction électrique, à chacune de la première et de la seconde prises de tension,

que, pour la saisie d'un potentiel de référence et pour l'alimentation du dispositif de mesure (7) en une alimentation de tension symétrique, une troisième prise de tension (3, 9) est prévue à une électrode située, dans le circuit de série, entre les électrodes extérieures,

que, pour la mesure de la tension totale de la batterie est prévu un circuit de mesure (4) qui est relié, avec conduction électrique, à ses entrées de mesure, avec la première et avec la seconde prises de tension et qui présente un transducteur de niveau et/ou un additionneur,

que, pour produire un signal d'état de la batterie, le dispositif de mesure (7) présente un élément à valeur de seuil (5) relié à la sortie du circuit de mesure (4), avec au moins une valeur de seuil,

que le circuit de mesure (4) et l'élément à valeur de seuil (5) présentent chacun une entrée pour un point de référence qui est relié, avec conduction électrique, à la troisième prise de tension (3, 9),

que, pour leur alimentation en une tension symétrique par rapport à la troisième prise de tension (3), le circuit de mesure (4) et l'élément à valeur de seuil (5) sont reliés, avec conduction électrique, à chacune de la première et de la seconde prises de tension.

2. Appareil alimenté par batterie selon la revendication 1, caractérisé par le fait

que, pour réaliser la synthèse, du point de vue technique de circuit, d'un additionneur, le circuit de mesure (4) présente quatre résistances ($R_1$, $R_2$, $R_3$, $R_4$) ainsi qu'un amplificateur opérationnel (OP) avec une entrée P (+) une entrée N (-), une sortie (11) et deux entrées d'alimentation en tension,

que la première résistance ($R_1$) relie l'entrée N (-) de l'amplificateur opérationnel (OP) avec la première prise de tension (8),

que, pour produire un rétrocouplage, la seconde résistance ($R_2$) relie l'entrée N (-) de l'amplificateur opérationnel (OP) avec sa sortie (11),

que la troisième résistance ($R_3$) relie l'entrée P (+) de l'amplificateur opérationnel (OP) avec la troisième prise de tension (9),

que la quatrième résistance ($R_4$) relie l'entrée P (+) de l'amplificateur opérationnel (OP) avec la seconde prise de tension (10),

que les entrées d'alimentation en tension de l'amplificateur opérationnel sont reliées avec la première (8) et avec la seconde (10) prises de tension,

que, pour remplir la condition relative aux coefficients, le rapport entre les valeurs de la seconde ($R_2$) et la première ($R_1$) résistances est sensiblement identique au rapport entre les valeurs de la troisième ($R_3$) et de la quatrième ($R_4$) résistances.

3. Appareil alimenté par batterie selon la revendication 1, caractérisé par le fait

que le circuit de mesure (4) présente un amplificateur pour l'adaptation de son signal de sortie à l'élément à valeur de seuil (5) et présente un transducteur de niveau pour la saisie de la totalité de la variation de la tension que fournit la batterie ainsi que pour l'adaptation à l'amplificateur,

que le transducteur de niveau est essentiellement constitué d'une diode Zener ($D_2$) et une première résistance ($R_3$), la cathode (K) de la diode Zener ($D_Z$) étant reliée à la première prise de tension (8) et la première résistance ($R_5$) reliant l'anode (A) de la diode Zener ($D_Z$) à la seconde prise de tension (10),

que, pour la saisie de la totalité de la variation de la tension que fournit la batterie et pour l'adaptation à l'entrée dans l'amplificateur est prévu un transistor (T) monté dans le circuit émetteur, la base (B) étant reliée à l'anode (A) de la diode Zener ($D_Z$) et l'émetteur (E) étant relié, par l'intermédiaire d'une seconde résistance ($R_6$), à la seconde prise de tension (10),

que l'amplificateur est essentiellement consti-

tué d'une troisième ($R_7$), d'une quatrième ($R_8$) et d'une cinquième ($R_9$) résistances, ainsi que d'un amplificateur opérationnel ($OP_2$) présentant une entrée N (-), une entrée P (+), une sortie ainsi que deux entrées d'alimentation en tension,

que l'entrée N (-) de l'amplificateur opérationnel (OP2) est reliée, par l'intermédiaire de la troisième résistance ($R_7$), au collecteur (C) du transistor (T),

que l'entrec P (+) de l'amplificateur opérationnel (OP2) est reliée, par l'intermédiaire de la quatrième résistance ($R_8$), à la troisième prise de tension (9),

que, pour obtenir un rétrocouplage, la sortie de l'amplificateur opérationnel (OP2) est reliée, par l'intermédiaire de la cinquième résistance $R_9$), à l'entrée N (-),

que les entrées d'alimentation en tension de l'amplificateur opérationnel (OP2) sont reliée; à la première (8) et à la seconde 10) prises de tension.

4. Appareil alimenté par batterie selon l'une des revendications précédentes, caractérisé par le fait qu'est prévu un boîtier (26) qui est relié, avec conduction électrique, à la troisième prise de tension (9).

5. Appareil alimenté par batterie selon l'une des revendications précédentes, caractérisé par le fait que le dispositif de mesure (7) présente un organe d'émission/réception pour la transmission, sans fil ou avec fil, du signal d'état de la batterie à un organe de réception situé à l'extérieur de l'appareil et/ou pour la réception d'un signal d'activation provenant d'un organe d'émission qui se trouve à l'extérieur de l'appareil.

6. Appareil alimenté par batterie selon l'une des revendications précédentes, caractérisé par le fait que le récepteur électrique (6) présente, pour influencer sa consommation de courant en fonction de l'état de charge de la batterie, un organe de commande à l'entrée duquel arrive le signal d'état de la batterie.

7. Appareil alimenté par batterie selon l'une des revendications précédentes, caractérisé par le fait

que la batterie présente un boîtier (26), hermétiquement étanche à l'état monté, avec deux cellules électriquement reliées par une cloison (35) pour donner un circuit de série, mais séparées dans l'espace,
que chaque cellule contient un paquet d'électrodes qui présente des plaques d'anode (16, 17, 18, 19, 20, 21, 22, 23, 33, 34, 35, 36) et des plaques de cathode (12, 13, 31, 32) plates ou incurvées, alternativement disposées l'une à côté de l'autre,
que, dans les deux cellules, les plaques de cathode (12, 13, 31, 32) sont reliées l'une à l'autre, avec conduction électrique, par un connecteur de cathodes (14, 15) et les plaques d'anode (16, 17, 18, 19, 20, 21, 22, 23, 33, 34, 35, 36) sont reliées l'une à l'autre, avec conduction électrique, par un connecteur d'anodes (24, 25, 40, 46),
que le connecteur d'anodes (25, 40) de la première cellule est relié, avec conduction électrique, à la première prise de tension (27) et le connecteur de cathodes (15) de la seconde cellule est relié, avec conduction électrique, à la seconde prise de tension (28),
que le connecteur d'anodes (24) de la seconde cellule et le connecteur de cathodes (14) de la première cellule sont reliés, avec conduction électrique, à la cloison (9), au boîtier (26) et à la troisième prise de tension (9).

8. Appareil alimenté par batterie selon l'une des revendications précédentes, caractérisé par le fait que, pour les isoler à l'égard du boîtier (26) et de la cloison (39), les paquets d'électrodes sont empilés dans une poche constituée de plastique.

9. Appareil alimenté par batterie selon l'une des revendications précédentes, caractérisé par le fait que le boîtier présente deux demi-coques (37, 43).

10. Appareil alimenté par batterie selon l'une des revendications précédentes, caractérisé par le fait que les plaques de cathode (12, 13, 31, 32) contiennent un mélange de $CrO_x$, x valant entre 2,5 et 2,7, ou $CrO_x$ et $MnO_2$ avec $PbCrO_4$, $PbMoO_4$ ou PbO ou un mélange de $MnO_2$ avec PbO.

11. Appareil alimenté par batterie selon l'une des revendications précédentes, caractérisé par le fait que les plaques de cathode (12, 13, 31, 32) présentent à la surface un revêtement qui contient un liant et un additif conducteur.

12. Appareil alimenté par batterie selon l'une des revendications précédentes, caractérisé par le fait que les plaques de cathode (12, 13, 31, 32) présentent une porosité de 30 à 50%.

13. Appareil alimenté par batterie selon l'une des revendications précédentes, caractérisé par le fait que les plaques d'anode (16, 17, 18, 19, 20, 21, 22, 23, 33, 34, 35, 36) sont essentiellement constituées de lithium.

14. Appareil alimenté par batterie selon l'une des revendications précédentes, caractérisé par le fait que la batterie contient un électrolyte qui est constitué de 20 à 60% de carbonate d'éthylène (EC) et de 5 à 20% de carbonate de propylène (PC) et 30 à 70% de diméthoxyéthane (DME).

15. Appareil alimenté par batterie selon l'une des revendications précédentes, caractérisé par le fait que l'électrolyte contient LiClO4 dans une concentration de 1,0 à 1,5 mole/litre.

16. Appareil alimenté par batterie selon l'une des revendications précédentes, caractérisé par le fait que le boîtier (26) est essentiellement constitué d'acier CrNi, de molybdène, de titane ou de nickel.

Fig.1

Fig.2

Fig.3

Fig.4

Fig.5